# EUROPEAN PATENT SPECIFICATION

(11) **EP 1 088 842 B1**
(45) Date of publication and mention of the grant of the patent: **07.09.2005**
(21) Application number: 00903966.0
(22) Date of filing: 16.02.2000
(51) Int. Cl.: C08G 61/12, C07D 333/08

(54) **OPTICALLY ACTIVE POLYTHIOPHENE**
OPTISCH AKTIVE POLYTHIOPHENE
POLYTHIOPHENE OPTIQUEMENT ACTIF

(30) Priority: 11.03.1999 JP 6569399
(43) Date of publication of application: 04.04.2001
(73) Proprietor: Japan Science and Technology Agency, Saitama 332-0012 (JP); NIPPON TELEGRAPH AND TELEPHONE CORPORATION, Tokyo 100-8116 (JP)
(72) Inventor: Fujiki, Michiya, Kanagawa 243-0036 (JP); Nakashima, Hiroshi, Zama-shi, Kanagawa 228-0011 (JP); Koe, Julian R., Atsugi-shi, Kana gawa 243-0036 (JP); Morita, Masao, Kanagawa 243-0036 (JP); Tamoto, Hiromi, Isehara-shi, Kanagawa 259-1117 (JP)
(74) Representative: Calamita, Roberto
(86) International application number: PCT/JP2000/000851
(87) International publication number: WO 2000/053659

(56) References cited:
- JP-A- 10 048 678
- DATABASE CA [Online] CHEMICAL ABSTRACTS SERVICE, COLUMBUS, OHIO, US; FUJIKI, MICHIYA ET AL: "UV-visible, circular dichroism, and fluorescence spectra of polythiophenes with (S)-2-methyloctyl side chains" retrieved from STN Database accession no. 130:352906 CA XP002184176 & POLYM. PREPR. (AM. CHEM. SOC., DIV. POLYM. CHEM.) (1999), 40(1), 523-524 , 1999,
- DATABASE CA [Online] CHEMICAL ABSTRACTS SERVICE, COLUMBUS, OHIO, US; LANGEVELD-VOSS, B. M. W. ET AL: "Main-chain chirality of regioregular polythiophenes" retrieved from STN Database accession no. 125:222885 CA XP002184177 & POLYM. PREPR. (AM. CHEM. SOC., DIV. POLYM. CHEM.) (1996), 37(2), 499-500 , 1996,
- DATABASE CA [Online] CHEMICAL ABSTRACTS SERVICE, COLUMBUS, OHIO, US; KOTKAR, DILIP ET AL: "Chiral conducting poly(3-alkylthiophenes): spectroscopic and electrochemical properties" retrieved from STN Database accession no. 117:192676 CA XP002184178 & FRONT. POLYM. RES., [PROC. INT. CONF.], 1ST (1991), 407-11. EDITOR(S): PRASAD, PARAS N.;NIGAM, JAI KRISHNA. PUBLISHER: PLENUM, NEW YORK, N. Y. , 1991,
- DATABASE CA [Online] CHEMICAL ABSTRACTS SERVICE, COLUMBUS, OHIO, US; KOTKAR, DILIP ET AL: "Towards chiral metals. Synthesis of chiral conducting polymers from optically active thiophene and pyrrole derivatives" retrieved from STN Database accession no. 109:171031 CA XP002184179 & J. CHEM. SOC., CHEM. COMMUN. (1988), (14), 917-18 , 1988,

## Description

The present invention concerns optically active polythiophenes. More specifically, the present invention concerns novel optically active polythiophenes useful as functional polymers such as polymeric semiconductors, polymeric conductors, electrochromic materials, electroluminescent materials, and nonlinear optical materials.

In recent years attention has been focused on polythiophenes as functional polymer materials, and studies directed towards the application of such materials as polymeric semiconductors, polymeric conductors, electrochromic materials, electroluminescent materials, and nonlinear optical materials have been reported. These conventional polythiophenes have absorption bands in the visible region of about 450 to 600 nm based on the π-conjugated main chain.

However, in practice, a broader absorption band is desirable, and in this sense the characteristics of conventional polythiophenes have had their limits. For example, to apply polythiophenes as luminescent materials, the absorption band related to the main chain should cover a broad region from ultraviolet to near infrared.

On the other hand, polymers such as poly(diphenylpyridylmethylmethacrylate), synthesized using sparteine-butyllithium as an initiator (e.g. Y. Okamoto et al., J. Am. Chem. Soc., *103*, 6971 (1981)), whose main chain construct a one-directional helix are known as polymer materials which recognize enantiomers. These organic polymers having one-directional helices are actually on the market, as column materials for high-performance liquid chromatography, supported on the surface of silica gel, which allows the separation and analysis of optical isomers (Daicel Chemical Industries, Ltd., Chiralpak-OT and Chiralpak-OP).

However, these poly(triphenylmethylmethacrylate)-based materials have a serious disadvantage, in that they lose their enantiomer recognition ability once the side-chain section which fix the main chain helices are released. Hence, if characteristics such as the high resistance to hydrolysis and solvolysis of the helical main chain and the hydrocarbon side chains could be utilized, polythiophenes may be applied as column materials for high-performance liquid chromatography (HPLC) and gas chromatography (GC) which are performed under dark deoxygenated conditions. That is, these materials may be expected to show resistance when used repeatedly for a long period of time.

Also, most column materials presently used for enantiomer recognition, which can be used in separation and analysis by HPLC under reversed phase conditions, are those derived from biological substances most of which are alkyl derivatives containing amide bonds, causing them to deteriorate by hydrolysis. If column materials could be derived from thiophene-based materials containing optically active substituents, or if optically active polythiophenes with helices in the main chain could be supported on carriers, new uses of such materials as column materials for HPLC and GC may be developed.

However, contrary to such anticipations, polythiophenes showing the required performances described above have not been known so far.

The present invention has been carried out under the above circumstances, and its objective is to overcome the limits of conventional technology, to provide novel optically active polythiophenes with strong absorption characteristics due to the main chain in the ultraviolet to visible region, particularly those which exhibit strong main chain absorption characteristics in the ultraviolet-visible region, in solutions at room temperature, and show optical activity by itself, and thiophene monomers which provide such optically active polythiophenes.

In order to accomplish the above objectives, the present invention provides, firstly, optically active polythiophenes represented by the following formulas (A2) and (B2):

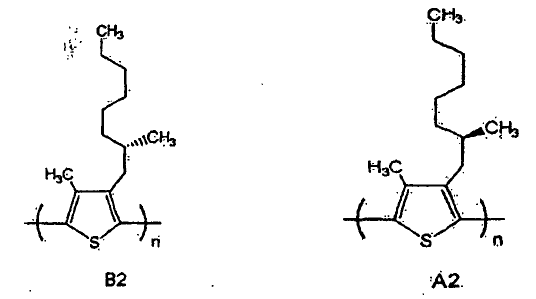

Secondly, the invention provides an optically active polythiophene represented by the following formula (A3):

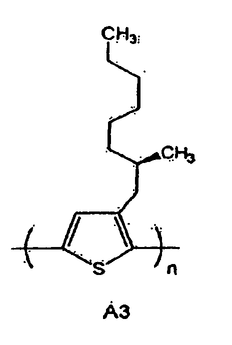

Furthermore, the present invention provides, as a monomer which provides the polythiophenes of the first invention optically active thiophenes represented by the following formulas (A6) and (B6):

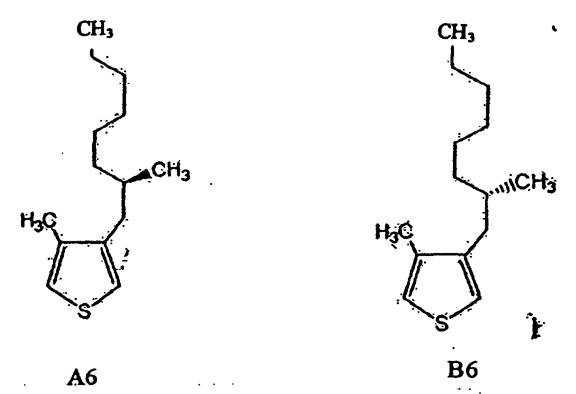

And fourthly, the invention provides an optically active thiophene represented by the following formula (A7):

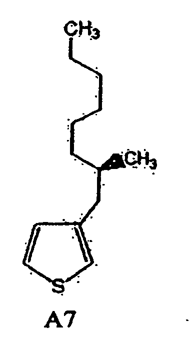

In the accompanying drawings:
Fig. 1 shows the ultraviolet-visible absorption and the circular dichroism spectrum (in tetrahydrofuran, 25°C) of poly[{3-methyl-4-(S)-2-methyloctyl}thiophene] prepared in Example 3.
Fig. 2 shows the ultraviolet-visible absorption spectrum and the circular dichroism spectrum (in tetrahydrof uran, 25°C) of poly[3-(S)-2-methyloctylthiophene] prepared in Example 4.
Fig. 3 shows the ultraviolet-visible absorption spectrum and the circular dichroism spectrum (in tetrahydrofuran, -79°C) of poly[3-(S)-2-methyloctylthiophene] prepared in Example 4.
Fig. 4 is the ¹³C-NMR spectrum (CDCl₃, 30°C) of poly[3-(S)-2-methyloctylthiophene] prepared in Example 4.
Fig. 5 shows the spectrum that follows Fig. 4.

The present invention provides thiophene homopolymers which contain chiral alkyl groups comprising a branch-structure at the β-position, and thiophene monomers which contain chiral alkyl groups having a branch-structure at the β-position and provide the homopolymers described above. Embodiments are further illustrated below.

The optically active polythiophenes of the present invention are those represented by the formulas (A2), (A3) and (B2) described above. The monomers producing these optically active polythiophenes are those represented by the formulas (A6), (A7) and (B6) described above.

For the optically active polythiophenes of the present invention, monomers may be produced by deriving chiral alkyl halides from commercially available chiral alcohols having structures corresponding to the β-branched alkyl groups mentioned above, which may be further derived to provide optically active alkyl-substituted thiophenes.

To polymerize these monomers, the branch point at the β-position prevents racemization and rearrangement during oxidative condensation reaction. In addition, because the steric hindrance between monomers during condensation reaction is comparatively small, optically active polythiophene chains with high molecular weights may be easily produced. The present inventors have found that polythiophene main chains with β-chiral carbons, particularly β-branched chiral alkyl groups, and more preferably for its solubility, a (S)-2-methyloctyl group, prove effective in providing optically active polythiophenes in which the direction of the helices is biased to one direction.

In the present invention, by selecting proper substituent groups for the side chains, fundamental substances suitable for the studies of basic optical and electrical properties of ideal one-dimensional semiconductors and quantum small wire structures with no chain interaction as in conventional optically active polythiophenes may be provided. In addition, the optically active polythiophenes of the present invention may also be used as a new type of enantiomer-recognizing material.

The number of repeating units (n) in the above general formulas may be 10 to 100,000 in the present invention.

The embodiments of the invention are further illustrated by the following examples. Of course, the following examples are not to be construed as limiting the invention.

### Examples

### Synthesis of Substituted Thiophenes

### Example 1 3-Methyl-4-(S)-2-methyloctylthiophene

8.3 g of (S) -2-methyloctyl bromide and 1.2 g of magnesium were reacted in 25 ml dry ether to prepare a Grignard reagent. 0.50 g of Nickel (0) bis-(diphenylphosphinopropane) dichloride was added to the reagent solution and 5.9 g of 3-methyl-4-bromothiophene was slowly added dropwise to the solution at 40°C. After reacting for 6 hours in total, the product obtained was decomposed by treatment with water and dilute hydrochloric acid, washed repeatedly with brine, and then neutralized to obtain the title compound in a yield of 4.0 grams (54%). B.p. 101-106°C/1.0 mmHg; [α]D (24°C, neat) -0.02°; ¹³C-NMR (CDCl₃, 30°C) 141.115, 137.197, 121.108, 120.694, 37.059, 36.675, 33.446, 31.960, 29.633, 27.154, 22.717, 19.684, 14.655, and 14.128 ppm.

### Example 2 3-(S)-2-Methyloctylthiophene

21 g of (S)-2-methyloctyl bromide and 2.7 g of magnesium were reacted in 75 ml of dry ether to prepare a Grignard reagent. The reagent was slowly added dropwise in 100 ml of a tetrahydrofuran solution of 0.40 g of nickel(0)bis(diphenylphosphinöpropane)dichloride and 13.0 g of 3-bromothiophene at 40°C. After reaction for a total of 6 hours, the reaction product was decomposed by treatment with water and dilute hydrochloric acid, washed repeatedly with brine, and neutralized to obtain the title compound in a yield of 9.1 g (54%). Bp 101-106°C/ 1.0mmHg; [α]D (24°C, neat) -0 .05°; ¹³C-NMR (CDCl₃, 30°C) 141.851, 128.739, 124.790, 120.659, 37.904, 36.732, 34.374, 32.013, 31.960, 29.616, 27.119, 22.713, 19.588, and 14.124 ppm.

### Synthesis of Optically Active Polythiophenes

### Example 3 Poly[{3-methyl-4-(S)-2-methyloctyl}thiophene]

After thoroughly drying, degassing, and replacing the inside of a reaction vessel with argon gas, a solution of 0.9 g of [3 -methyl-4-(S)-2-methyloctyl]-thiophene in 10 ml of chloroform was added dropwise to a solution of 2.5 g of ferric chloride in 50 ml of chloroform, and allowed to react with stirring at room temperature for one day. The reaction mixture solution was dissolved in chloroform and reduced with an aqueous hydrazine. Solvents were removed in vacuum and the residue was washed with acetone for two days while heating. The title polymer was obtained in a yield of 0.36 g (40%). The weight-average molecular weight was 107,000 and the number-average molecular weight was 3200:

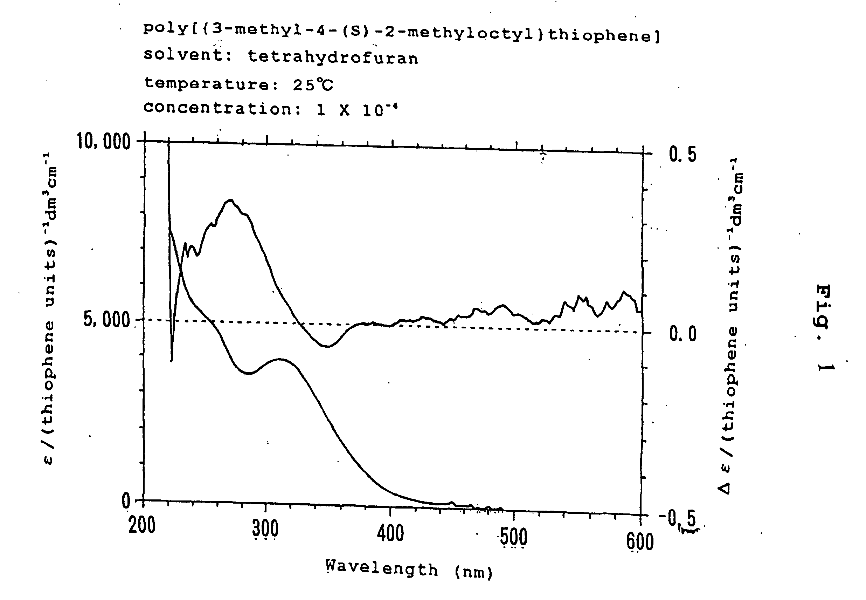

Fig. 1 shows the ultraviolet-visible absorption spectrum and the circular dichroism spectrum (in tetrahydrofuran, 25°C) of poly[{3-methyl-4-(S)-2-methyloctyl}thiophene] prepared in Example 3.

In Fig. 1 Cotton CD absorption bands of 270 nm (positive) and 350 nm (negative) were observed at 25°C.

### Example 4 Poly[3-(S)-2-methyloctylthiophene]

After thoroughly drying, degassing, and replacing the inside of a reaction vessel with argon gas, a solution of 1.05 g of 3-(S)-2-methyloctylthiophene in 10 ml of chloroform was added dropwise to a solution of 1.9 g of ferric chloride in 50 ml of chloroform and stirred at room temperature for one day. The reaction mixture solution was dissolved in chloroform and reduced with an aqueous hydrazine. Solvents were removed in vacuum and the residue was washed with acetone for two days while heating. The title polymer was obtained in a yield of 0.32 g (31%). The weight-average molecular weight was 123, 000 and the number-average molecular weight wäs 23,000 .

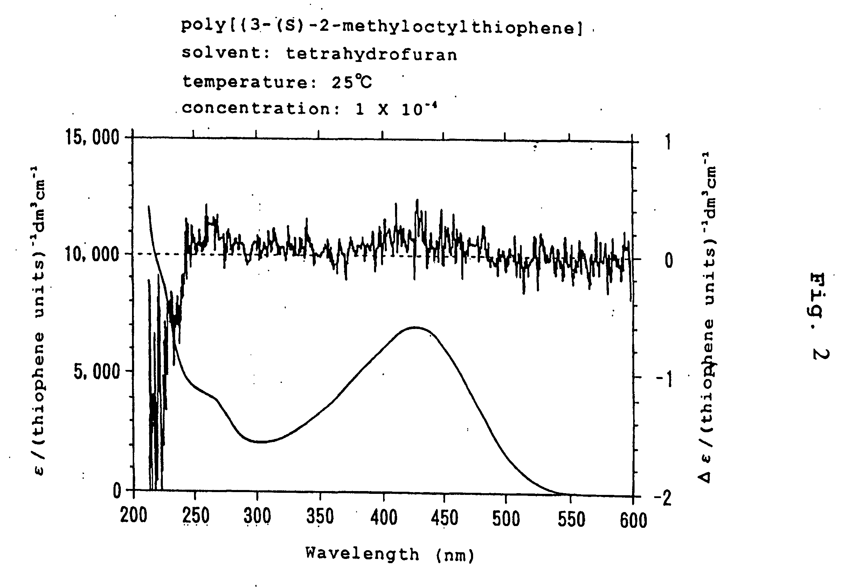

Fig. 2 shows the ultraviolet-visible spectrum and the circular dichroism spectrum (in tetrahydrofuran, 25°C) of poly[3-(S)-2-methyloctylthiophene] prepared in Example 4. Fig. 2 reveals that no Cotton CD absorption band is observed at 25°C.

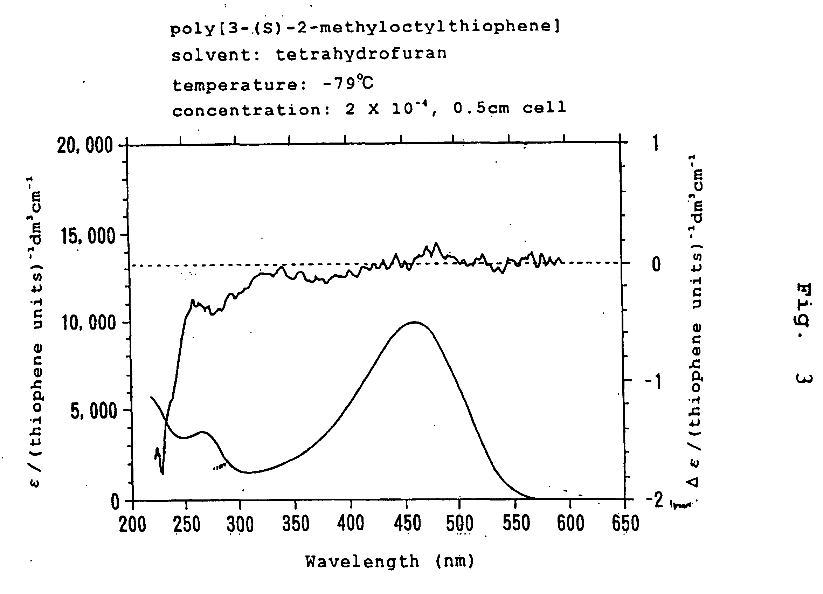

Fig. 3 shows the visible absorption spectrum and the circular dichroism spectrum (in tetrahydrofuran) at -78°C. Very weak Cotton CD absorption bands were observed at about 475 nm, about 380 nm, and about 270 nm as compared with the spectra for poly [bis{3,4-(S)-2-methyloctyl}thiophene] and poly[{3-methyl-4-(S)-2-methyloctyl}thiophene].

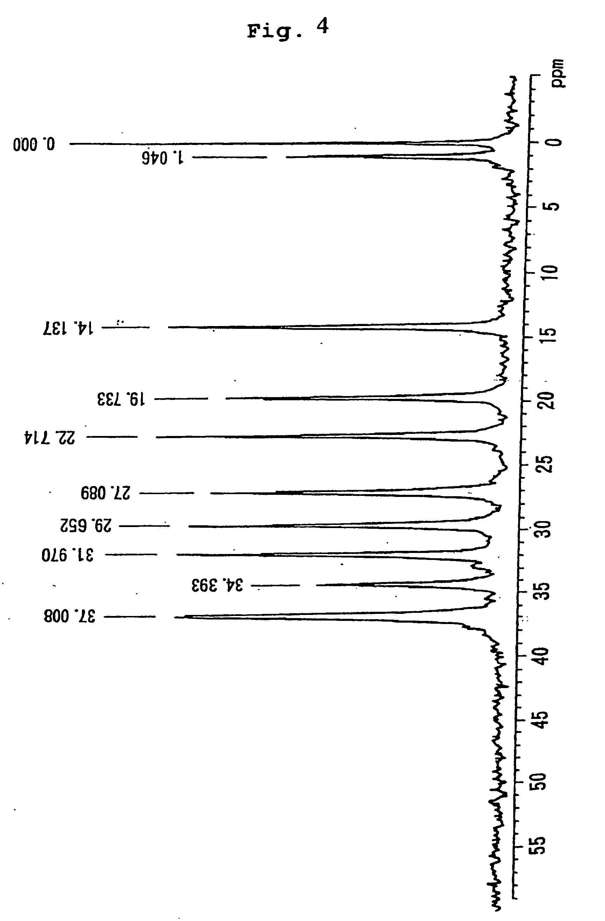

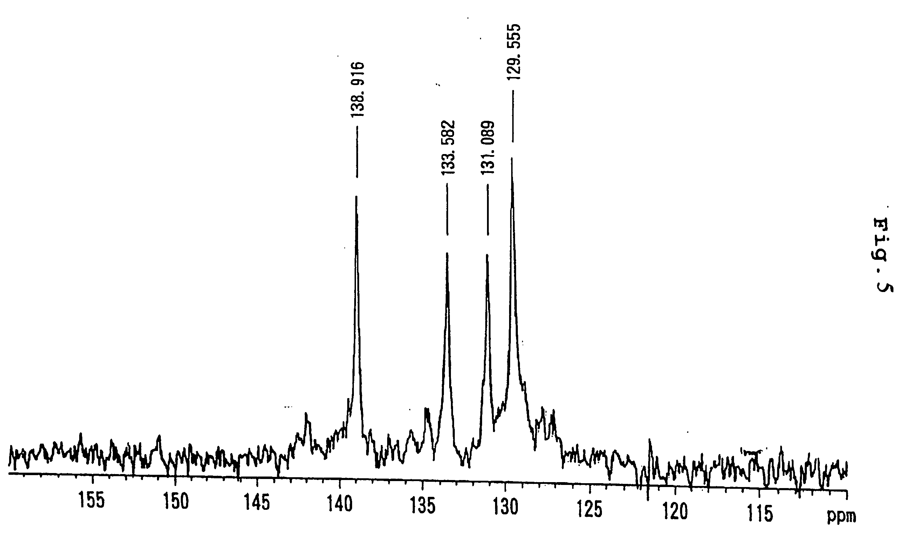

Fig. 4 and Fig. 5 show the ¹³C-NMR spectra (CDCl₃, 30°C).

As illustrated above in detail, by using monomers comprising alkyl groups with branch-structures at the β-position, especially the two thiophene monomers comprising (S)-2-methyloctyl groups, the present invention easily provides solvent-soluble optically active polythiophenes where the direction of the helices is oriented to one direction. Furthermore, by properly selecting side chain substituents, the optically active polythiophenes of the present invention may be extremely useful compared to conventional optically non-active polythiophenes, as fundamental model substances for the study of optical and electrical properties of ideal one-dimensional semiconductors with no interaction between chains, or of ultimate quantum small wire structure in an atomic level that may be realized in the near future. The polythiophenes of the present invention may be used as standard substances of polymers as well. These optically active polythiophenes may also be expected to serve as a new type of enantiomer-recognizing material.

## Claims

1. An optically active polythiophene represented by the following formula (A3): 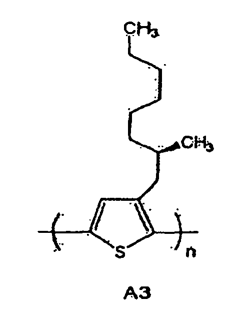 , wherein n represents the number of the repeating units selected from 10 to 100000.

2. An optically active thiophene represented by the following formula (A7):

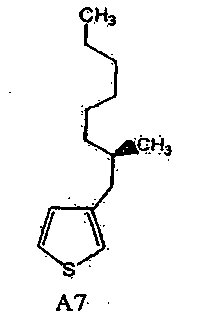

3. Optically active polythiophenes represented by either one of the following formulas (A2) or (B2): 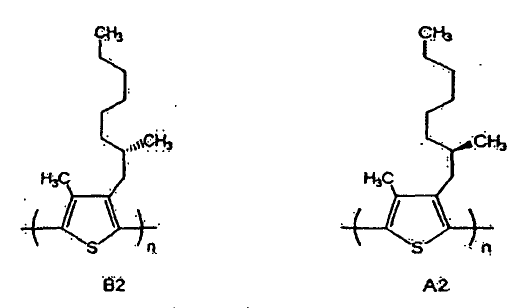 , wherein n represents the number of the repeating units selected from 10 to 100000.

4. Optically active thiophenes represented by either of the following formulas (A6) and (B6):

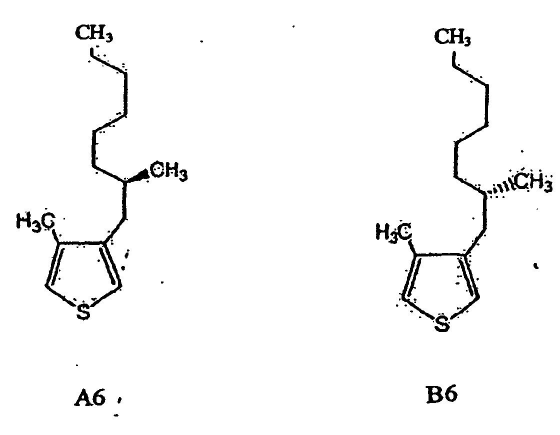

## Patentansprüche

1. Optisch aktives Polythiophen der folgenden Formel (A3), 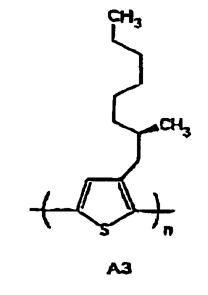 worin n für die Anzahl der Wiederholungseinheiten steht und unter 10 bis 100 000 ausgewählt ist.

2. Optisch aktives Thiophen der folgenden Formel (A7):

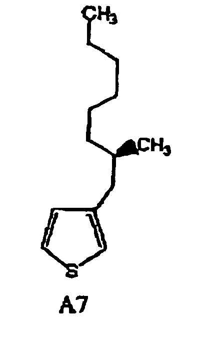

3. Optisch aktive Polythiophene der folgenden Formel (A2) oder (B2): 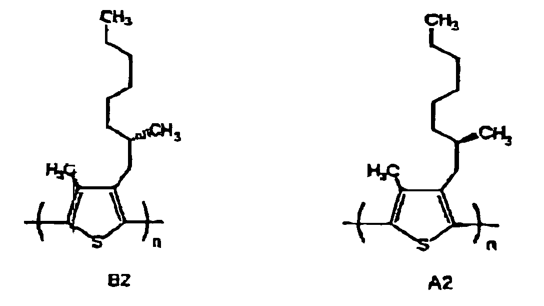 worin n für die Anzahl der Wiederholungseinheiten steht und unter 10 bis 100 000 ausgewählt ist.

4. Optisch aktive Polythiophene der folgenden Formel (A6) oder (B6):

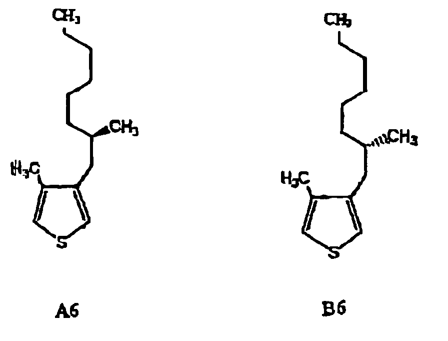

## Revendications

1. Polythiophène optiquement actif représenté par la formule suivante (A3) : 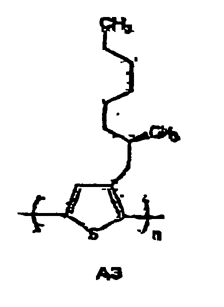 dans laquelle n représente le nombre de motifs répétés sélectionnés de 10 à 100 000.

2. Thiophène optiquement actif représenté par la formule suivante (A7) :

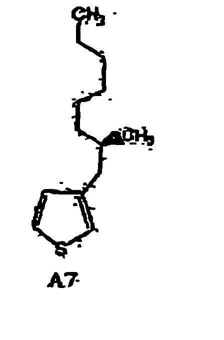

3. Polythiophènes optiquement actifs représentés par l'une des formules suivantes (A2) ou (B2) : 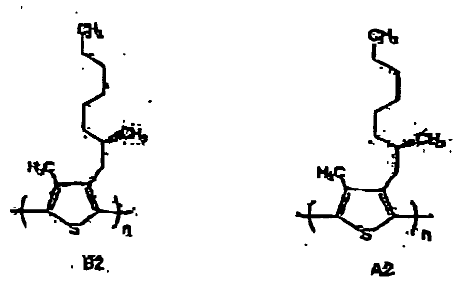 dans lesquelles n représente le nombre de motifs répétés sélectionnés de 10 à 100 000.

4. Thiophènes optiquement actifs représentés par l'une des formules suivantes (A6) et (B6):
